(19) Europäisches Patentamt
European Patent Office
Office européen des brevets

(11) **EP 4 397 328 A1**

(12) **EUROPEAN PATENT APPLICATION**
published in accordance with Art. 153(4) EPC

(43) Date of publication:
**10.07.2024 Bulletin 2024/28**

(21) Application number: 22864529.7

(22) Date of filing: **30.08.2022**

(51) International Patent Classification (IPC):
**A61L 27/04** (2006.01)    **A61L 27/06** (2006.01)
**A61L 27/12** (2006.01)    **A61L 27/30** (2006.01)
**A61L 27/42** (2006.01)    **A61L 27/50** (2006.01)

(52) Cooperative Patent Classification (CPC):
**A61L 27/04; A61L 27/06; A61L 27/12; A61L 27/30;
A61L 27/42; A61L 27/50**

(86) International application number:
**PCT/JP2022/032506**

(87) International publication number:
**WO 2023/032947 (09.03.2023 Gazette 2023/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2021 JP 2021140956**

(71) Applicant: **Maruemu Works Co., Ltd.
Osaka-shi, Osaka 5420086 (JP)**

(72) Inventors:
• **NARITA, Kengo**
 **Daitou-shi, Osaka 574-0015 (JP)**
• **YAMANAKA, Shigeru**
 **Daitou-shi, Osaka 574-0015 (JP)**

(74) Representative: **Meissner Bolte Partnerschaft
mbB
Widenmayerstraße 47
80538 München (DE)**

(54) **BIOCOMPATIBLE MATERIAL AND METHOD FOR MANUFACTURING SAME**

(57) The present invention provides a biocompatible material in which a hydrophilic substrate is protected by a metal film, the metal film being dissolved when the biocompatible material is used in a living body environment, enabling the protected hydrophilic substrate to be used in a state in which hydrophilicity is retained. The present invention provides a biocompatible material having: a biocompatible substrate that has a hydrophilic surface; and a metal film provided on the surface of the substrate, the metal film having the dissolution characteristic of dissolving in body fluids or simulated body fluids.

Fig. 3

EP 4 397 328 A1

**Description**

Technical Field

The present invention relates to a biocompatible material provided with a protective film, specifically a metal film, on a substrate, and the protective film, specifically the metal film, of the biocompatible material maintains the hydrophilicity of the substrate until the biocompatible material is used in a biological environment.

Specifically, the present invention relates to a biocompatible material comprising a biocompatible substrate that is hydrophilic; and a metal film provided on the substrate, the metal film having a dissolution property of dissolving in a predetermined liquid.

The present invention also relates to a method for producing the biocompatible material.

Background Art

The treatment of teeth by implants has attracted attention as a type of treatment method for tooth loss for young people to elderly people.

Since loosening is likely to occur after implantation due to insufficient adhesion between the screw portion of the root implant and the bone, there is a need to improve the adhesion between the screw portion and bone cells.

In addition, since the contact portion (abutment portion) between the root implant and the gingiva is prone to inflammation due to adhesion of bacteria, there is a demand for a technique for suppressing the adhesion of bacteria to the abutment portion.

Since the adhesiveness between an implant member and living tissue generally correlates with the hydrophilicity of the surface of the implant member, prior art techniques have been developed to control the hydrophilicity of the surface of the implant member.

Further, there is a close relationship between the surface roughness and hydrophilicity of the substrate. Since the contact area increases as the surface roughness increases, it becomes sensitive to changes in surface conditions.

Therefore, the rougher the surface roughness, the more difficult it is to maintain its hydrophilicity for a long period of time.

For example, Patent Document 1 discloses a technique for imparting an activated surface with superhydrophilicity by removing organic contaminants adhering to a titaniumbased biological implant with ultraviolet light immediately before surgery. However, a special device is required to irradiate the bioimplant with ultraviolet light at the surgical site, and the operation during surgery is complicated.

Further, Patent Document 2 discloses a technique of coating an implant hydrophilized by ultraviolet irradiation with a solution containing a drug. However, drugcontaining coatings tend to deteriorate when stored in the atmosphere.

Patent Document 3 discloses a technique in which a dental implant coated with a hydroxyapatite is sealed in a container, subjected to radiation sterilization, and stored, and immediately before surgery, water or an aqueous solvent is poured into the container and shaken, thereby to remove impurities attached to the surface of the hydroxyapatite coating at the surgical site. However, an increase in the number of procedures at the time of surgery is complicated, and there is a possibility that an unforeseen situation may arise because the impurity removal step is entrusted to the clinical site.

Patent Document 4 discloses a technique that maintains antibacterial properties and hydrophilicity for a long period of time by forming an oxide composed of titanium and iodine on the surface of the base material. However, the technique maintains hydrophilicity using the photocatalytic reaction of titanium oxide, and thus it cannot maintain hydrophilicity in a dark place such as inside a container.

Patent Document 5 discloses a technique for imparting antibacterial properties and hydrophilicity by directly fixing a hydrophilic organic compound on a metal surface. However, the technique directly electrodeposits polymers onto metals, and thus it is difficult to uniformly apply the polymer to complex shapes. In addition, since it is a soft polymer, local detachment and breakage cannot be avoided during screw insertion.

Patent Document 6 discloses a technique of a solution and a preservation kit that can maintain a hydrophilic state by anodization, hydrothermal treatment, ultraviolet irradiation, plasma irradiation, and the like. The above-described techniques show that the hydrophilic state can be maintained only in a solution, and thus, that it must be stored in a container containing the solution in order to maintain the hydrophilic state.

Patent Document 7 discloses a technique in which a Mgrich layer integrated with a substrate is provided on ceramics, and the film ensures hydrophilicity. Immediately after production, it exhibits superhydrophilicity and has a contact angle of 0°, but when exposed to the atmosphere, it gradually loses its hydrophilicity and reaches a contact angle of 40° or more in 5 days or more.

Patent Document 8 discloses a technique in which a Carich layer is provided on ceramics and hydrophilicity is ensured by the film. In this case as well, the film exhibits superhydrophilicity immediately after production and has a contact angle of 0°, but when exposed to the air, it gradually loses its hydrophilicity, and after several weeks, the contact angle reaches 40° or more. Note that the films of Patent Documents 7 and 8 are stable and insoluble because they are compounds. Thus, a storage method or a relatively short expiration date must be provided to maintain the hydrophilicity of the films.

Patent Document 9 discloses a technique relating to implants and coating layers such as calcium-containing magnesium and trace amounts of iron and nickel. However, since the technique contains insoluble $Mg_2Ca$, it may be taken into the body and cause harm. In addition, the coating layer in the technique does not dissolve completely, and the base material and the bone are adhered via the coating layer. Therefore, the hydrophilicity of the substrate is not related to its adhesion to the bone.

Prior Art Document

Patent Document

Patent Document 1: JP-A No. 2014-193249.
Patent Document 2: JP-A No. 2016-154935.
Patent Document 3: JP-A No. 2017-169821.
Patent Document 4: JP-A No. 2016-053195.
Patent Document 5: JP-A No. 2007-181515.
Patent Document 6: JP-A No. 2014-014487.
Patent Document 7: JP Patent No. 6095653.
Patent Document 8: JP Patent No. 6154806.
Patent Document 9: JP Patent No. 5705163.

Summary of Invention

Problems to be solved by the Invention

Various techniques for hydrophilizing biocompatible materials have been proposed as the above-mentioned prior art. However, there has been a problem that it is necessary to store it in a special container containing a solution to protect hydrophilicity, or to go through a process to impart hydrophilicity at the surgical site, in order to exhibit a highly hydrophilic state during surgical use.

Accordingly, an object of the present invention is to provide a technique that does not require a storage container or solution for maintaining hydrophilicity and does not require a step of imparting hydrophilicity at the surgical site.

Specifically, an object of the present invention is to provide a biocompatible material, in which a hydrophilic substrate is protected with a protective film, specifically a metal film; the protective film, specifically, the metal film, is dissolved; and the protected substrate having hydrophilicity can be used while retaining its hydrophilic properties upon using the biocompatible material in a biological environment.

Another object of the present invention is, other than or in addition to the above objects, to provide a method for producing the above biocompatible material.

Means for Solving Problems

The present inventors have found the following inventions:

<1> A biocompatible material comprising a biocompatible substrate having a hydrophilic surface; and a metal film provided on the surface of the substrate, the metal film having the dissolution property of dissolving in body fluid or simulated body fluid.
<2> In the above item <1>, when the biocompatible material is brought into contact with the body fluid or simulated body fluid, the metal film may dissolve and the biocompatible substrate having a hydrophilic surface may be exposed.
<3> In the above item <1> or <2>, the bodily fluid may be at least one selected from the group consisting of blood, lymph, bone marrow fluid, and tissue fluid, and the simulated bodily fluid may be at least one selected from the group consisting of physiological saline, phosphatebuffered saline (PBS), Hank's balanced salt solution (HBSS), SBF solution, plasma solution, cell culture solution, Eagle (MEM) solution, DMEM solution, and serum medium.
<4> In any one of the above items <1> to <3>, the simulated bodily fluid may be Hank's balanced salt solution.
<5> In any one of the above items <1> to <4>, the hydrophilic property has a water droplet contact angle of 90° or less, preferably 50° or less, more preferably 30° or less, most preferably 20° or less.

<6> In any one of the above items <1> to <5>, the metal film may comprise at least one metal selected from the group consisting of Mg, Ca, Zn and Fe.

<7> In any one of the above items <1> to <6>, the metal film may comprise magnesium and optionally calcium, and calcium may have 0 to 40 % by weight, preferably 0.8 to 35 % by weight, more preferably 5 to 35 % by weight, further preferably 15 to 35 % by weight, most preferably 25 to 35 % by weight, where a total weight of magnesium and calcium is 100 % by weight.

<8> In the above item <7>, the metal film may be free from $Mg_2Ca$.

<9> In any one of the above items <1> to <8>, the metal film may have an amorphous portion.

<10> In any one of the above items <1> to <9>, the substrate may be at least one selected from the group consisting of pure titanium, a cobalt-chromium alloy, stainless steel, titanium alloys, zirconia, alumina, calcium phosphate and magnesia.

<11> In any one of the above items <1> to <10>, surface roughness Ra of the substrate may be 50 um or less, preferably 40 um or less, more preferably 30 um or less.

<12> In any one of the above items <1> to <11>, a shape of the biocompatible material may be one selected from the group consisting of a cylindrical shape, a truncated cone shape, a conical shape, a shape having a screw-shaped threaded portion in a part of the shape, a rectangular parallelepiped and a cube, a block shape having a partially inclined surface, and a wedge shape.

<13> In any one of the above items <1> to <12>, the biocompatible material may be one selected from the group consisting of an artificial bone material, an intraosseous fixture material, a dental implant material, an orthodontic anchor screw material, a medullary nail material, and an interbody fixation material.

<14> A method of producing a biocompatible material, comprising the steps of:

(A) preparing a biocompatible substrate;
(B) hydrophilizing the surface of the biocompatible substrate; and
(C) forming a metal film on the surface of the biocompatible substrate;

thereby, to produce the biocompatible material comprising a biocompatible substrate having a hydrophilic surface; and a metal film provided on the surface of the substrate.

<15> In the above item <14>, the step (C) may comprise

(C1) preparing a sputtering target comprised of the metal film precursor; and
(C2) sputtering by using the sputtering target, to form the metal film on the biocompatible substrate obtained in the step (B).

<16> In the above item <15>, the step (C1) may be (C1a) preparing the sputtering target comprised of magnesium and optional calcium,
the step (C2) may be (C2a) sputtering by using the sputtering target and setting a temperature of the biocompatible substrate obtained in the step (B) at 130°C or lower, preferably 90°C or lower, more preferably 60°C or lower, to form the metal film comprised of magnesium and optional calcium on the biocompatible substrate, wherein the metal film comprises 0 to 40 % by weight of calcium where a total weight of magnesium and calcium is 100 % by weight.

<17> In any one of the above items <14> to <16>, the step (B) may be at least one selected from the group consisting of acid treatment, blasting treatment, anodizing, hydrothermal treatment, ultraviolet irradiation, plasma irradiation, laser irradiation, radiation irradiation, and ion irradiation.

<18> In any one of the above items <14> to <17>, the step (B) may be ion irradiation, and an integral power at the ion irradiation may be 0.4 W·min/cm$^2$ or more, preferably 0.8 W·min/cm$^2$ or more, more preferably 2.5 W·min/cm$^2$ or more, further preferably 5 W·min/cm$^2$ or more, most preferably 5 to 32 W·min/cm$^2$.

<19> In any one of the above items <14> to <18>, the method may further comprise the step of (D) ion cleaning the surface of the biocompatible substrate after the step (B) and before the step (C).

Effects of the Invention

The present invention can provide a technique that does not require a storage container or solution for maintaining hydrophilicity and does not require a step of imparting hydrophilicity at the surgical site.

Specifically, the present invention can provide a biocompatible material, in which a hydrophilic substrate is protected with a protective film, specifically a metal film; the protective film, specifically, the metal film, is dissolved; and the protected substrate having hydrophilicity can be used while retaining its hydrophilic properties upon using the biocompatible material in a biological environment.

Further, other than or in addition to the above effect, the present invention can provide a method for producing the above biocompatible material.

Brief Description of Drawings

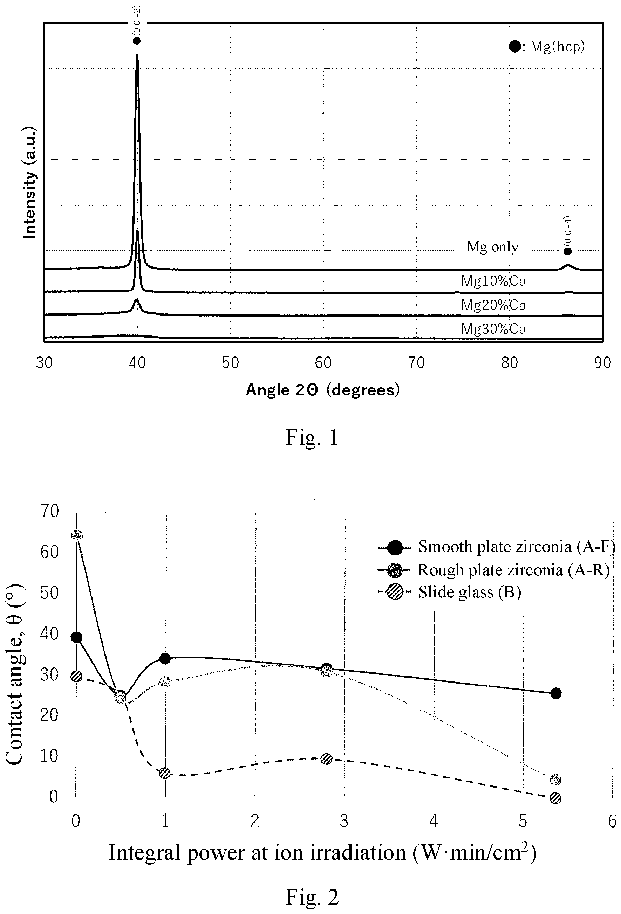

Fig. 1 is a graph showing X-ray diffraction profiles for an Mg film, an Mg10%Ca film, an Mg20%Ca film, and an Mg30%Ca film, each of which were obtained by subjecting ion irradiation to the glass substrate B, followed by sputtering using only magnesium (Mg), 10 % by weight (Mg10%Ca), 20 % by weight (Mg20%Ca) and 30 % by weight (Mg30%Ca) of an amount of Ca as a target.

Fig. 2 shows water droplet contact angles of surfaces of smooth plate zirconia A-F, rough plate zirconia A-R, and glass substrate B irradiated with ions at the integral power of 0, 0.495, 0.99, 2.85 or 5.36 $W \cdot min/cm^2$.

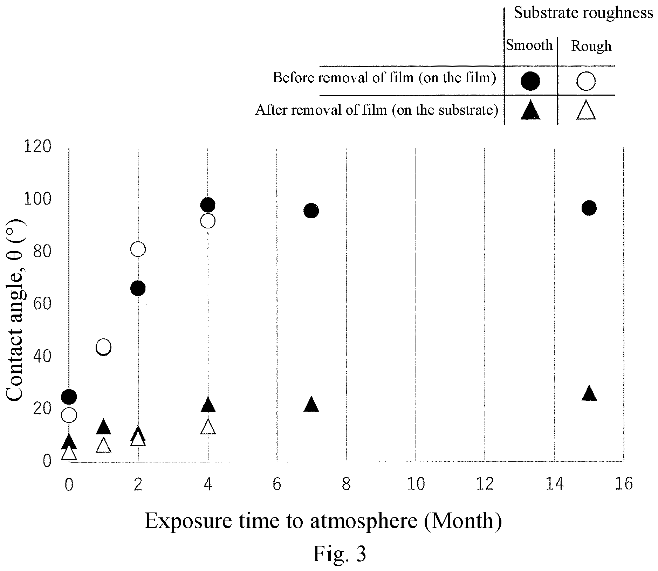

Fig. 3 shows water droplet contact angles immediately after removing the Mg30%Ca films, which were prepared by ion irradiation with an integral power of 0.99 $W \cdot min/cm^2$, followed by sputtering, coated on smooth plate zirconia A-F or A-F2, and stored for one week, 1 month, 2 months, 4 months, 7 months, and 15 months after the coating.

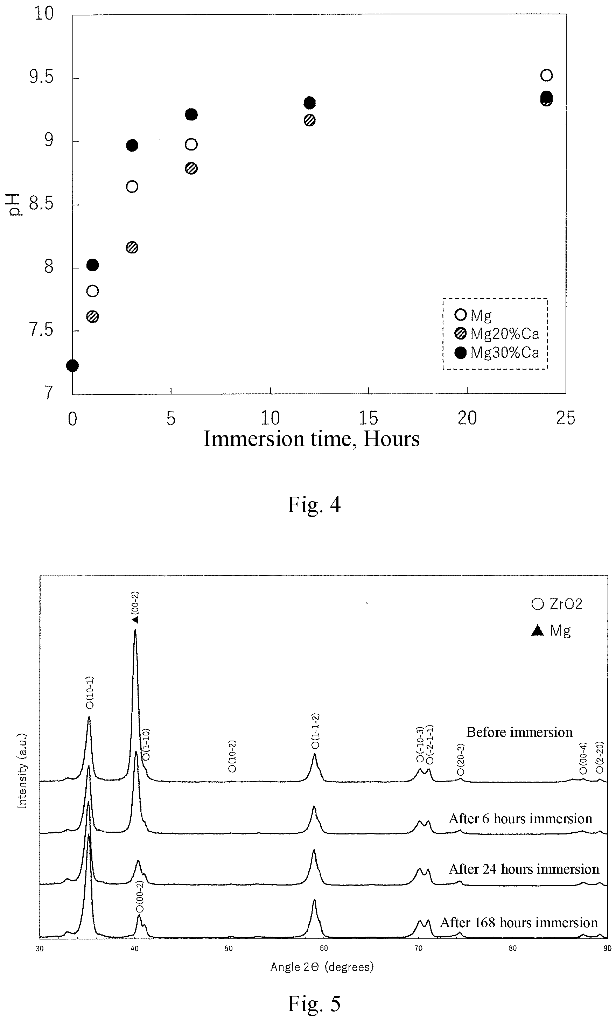

Fig. 4 is a diagram showing the pH measurement results for evaluating the dissolution rate in HBSS(-) immersion for smooth zirconia A-F2 with Mg film, Mg20%Ca film, and Mg30%Ca film.

Fig. 5 is a diagram showing the XRD measurement results of smooth zirconia A-F2 coated with an Mg film before being immersed in HBSS (-) and after being taken out after 6 hours, 24 hours, and 168 hours immersion.

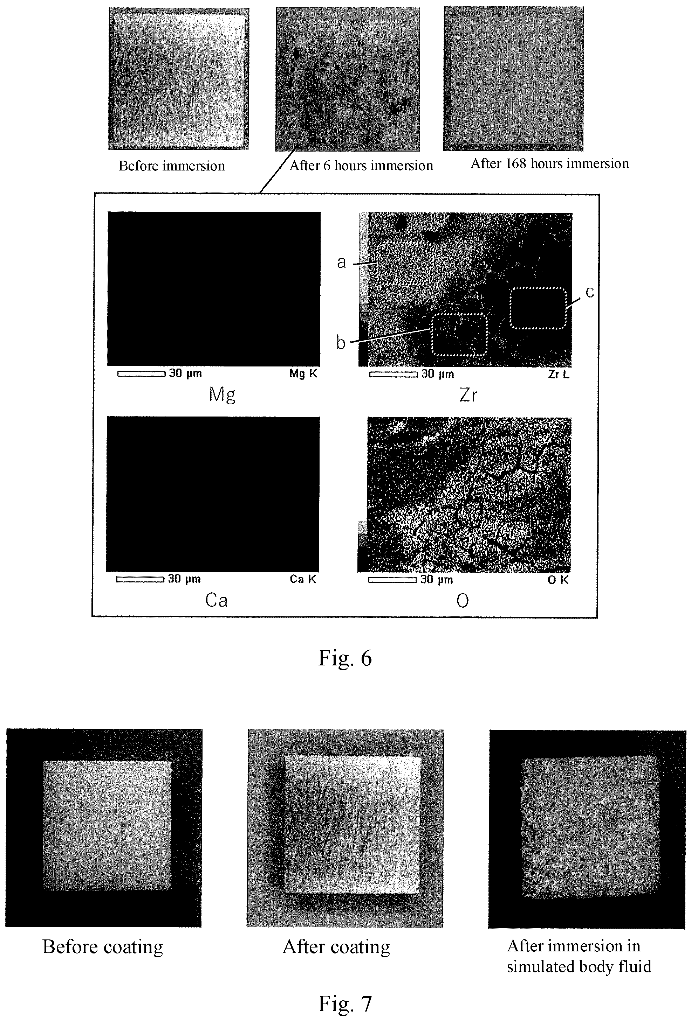

Fig. 6 shows appearance photographs of smooth zirconia A-F2 with a Mg30%Ca film before immersion in HBSS (-), taken out after 6 hours immersion, and after 168 hours immersion, and analysis results by EDX of the surface of the sample taken out after 6 hours immersion.

Fig. 7 shows a photograph of smooth plate zirconia A-F, a photograph of a sample in which smooth plate zirconia A-F was irradiated with ions at an integral power of 5.36 $W \cdot min/cm^2$, followed by sputtering to coat with a Mg30%Ca film, and a photograph of a sample in which a smooth plate zirconia A-F was irradiated with ions at an integral power of 5.36 $W \cdot min/cm^2$, followed by sputtering to coat with a Mg30%Ca film, immersed in a simulated body fluid (HBSS(+)), and then taken out after one week.

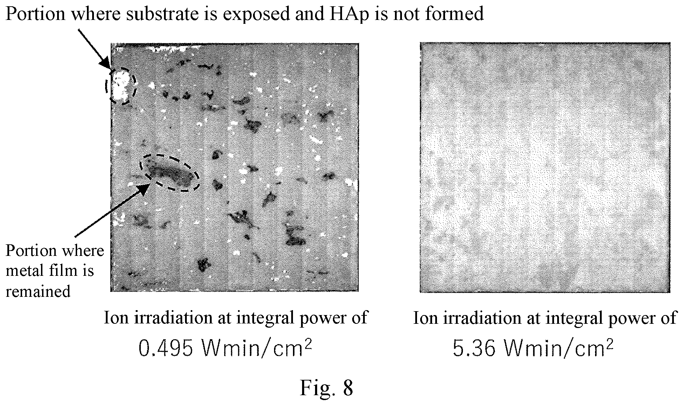

Fig. 8 shows an oblique light observation photograph taken with a microscope for a sample in which smooth plate zirconia A-F was irradiated with ions at an integral power of 0.495 $W \cdot min/cm^2$ and 5.36 $W \cdot min/cm^2$, then coated with a Mg30%Ca film by sputtering, immersed in a simulated body fluid (HBSS(+)), and taken out after one week.

Description of Embodiments

The invention described in the present application (hereinafter, may be abbreviated as the "present invention") is described hereinafter.

The present application provides a biocompatible material comprising a substrate having a hydrophilic surface; and a metal film provided on the surface of the substrate, the metal film having the dissolution property of dissolving in predetermined fluid.

Further, the present application provides a method for producing the biocompatible material.

<A biocompatible material>

The present application provides a biocompatible material comprising a biocompatible substrate having a hydrophilic surface; and a metal film provided on the surface of the substrate, the metal film having the dissolution property of dissolving in body fluid or simulated body fluid.

Furthermore, the term "biocompatibility" used herein means a characteristic of being maintained in a living body and not causing a biological safety problem.

<<A biocompatible substrate>>

The biocompatible material according to the present invention comprises a biocompatible substrate.

The biocompatible substrate has a hydrophilic surface. The hydrophilicity may be a water droplet contact angle of 90° or less, preferably 50° or less, more preferably 30° or less, and most preferably 20° or less.

Furthermore, the water droplet contact angle can be determined by a conventionally known method. For example, a droplet is dropped on the sample surface, and the angle formed by the sample surface and the droplet can be measured by the θ/2 method. More specifically, it can be measured 30 seconds after the droplet contacts the sample surface.

The biocompatible substrate is not particularly limited as long as it has the above-mentioned "biocompatibility". Examples may include, but are not limited to, metals such as pure titanium, cobalt-chromium alloys, stainless steel, titanium alloys and the like, and ceramics such as zirconia, alumina, calcium phosphate, magnesia and the like. The biocompatible substrate may be preferably pure titanium, titanium alloys and zirconia, more preferably ceramics, and further preferably zirconia. In the present invention, even if the biocompatible substrate is nonconductive ceramics, when the metal film and body fluid or simulated body fluid come into contact with each other, the metal film dissolves and the surface of the biocompatible substrate is exposed, resulting in hydrophilicity.

The biocompatible substrate may have a surface roughness (arithmetic mean roughness) Ra of 50 um or less, preferably 40 um or less, more preferably 30 um or less.

Surface roughness Ra can be determined by a conventionally known method, for example, according to JIS B 0601: 2013. In the standard, a reference length and an evaluation length are defined according to Ra value categories. For example, for $0.1 < Ra(um) \leq 2$, the reference length and evaluation length are 0.8 mm and 2.0 mm, respectively. In a case where the reference length cannot be measured continuously due to interference between the stylus and the object, the reference length in the category lower than the Ra value of the object is used, and the total number of times where the evaluation length is exceeded is measured, and then the average value can be measured as Ra.

<<A metal film>>

The biocompatible material according to the present invention comprises a metal film on a surface of the biocompatible substrate.

The metal film has dissolution property that dissolve in body fluids or simulated body fluids.

The metal film according to the present invention is biocompatible in that when the biocompatible material is brought into contact with a body fluid or a simulated body fluid, the biocompatible substrate and the metal film are not integrated, the metal film is dissolved, and the substrate, the surface of which is hydrophilic, may be exposed.

Examples of bodily fluids may include, but are not limited to, blood, lymph, bone marrow fluid, tissue fluid, and the like. In addition, examples of simulated body fluids may include, but not limited to, physiological saline, phosphate buffered saline (PBS), Hank's balanced salt solution (HBSS), SBF solution, plasma liquid, cell culture medium, Eagle (MEM) solution, DMEM solution, serum medium and the like.

As described above, the metal film according to the present invention has the property of dissolving in body fluids or simulated body fluids. Therefore, when a biocompatible material comprising the metal film on its surface is implanted in the body, the metal film is dissolved and the surface of the biocompatible material becomes hydrophilic. The surface of the substrate having hydrophilicity is exposed, exhibiting high adhesiveness to living tissue.

Furthermore, before using the biocompatible material, preferably immediately before use, the metal film of the present invention is brought into contact with a body fluid or a simulated body fluid to dissolve the metal film, thereby exposing the biocompatible substrate having a hydrophilic surface, to be possible to exhibit high adhesiveness to living tissue.

Alternatively, the metal film can be dissolved when the biocompatible material is used, preferably during use, so that the hydrophilic surface of the biocompatible substrate can exhibit the desired effect. Preferably, the metal film should have the dissolution properties of a) dissolving in 5% hydrochloric acid, and b) more preferably dissolving in Hank's balanced salt solution.

There are four types of Hank's balanced salt solutions, depending on whether they contain "Ca and Mg" and whether they contain "phenol red". Phenol red-free and "Ca and Mg"-free type of Hank's balanced salt solutions is preferred.

a) The dissolution property in 5% hydrochloric acid may satisfy one or both of the following i) and ii), for example, when 2 mg of the protective film is immersed in 50 ml of 5% hydrochloric acid:

i) The time when the concentration of the substance dissolved from the protective film becomes almost constant depending on the immersion time is 0.3 seconds or more, preferably 0.5 to 20 seconds, more preferably 0.5 to 10 seconds after immersion;
ii) The time when 2 mg of the protective film are substantially dissolved is 0.5 seconds or longer, preferably 1 to 60 seconds, more preferably 1 to 30 seconds after immersion.

Furthermore, the "substance dissolved from the protective film" depends on the components of the protective film. Moreover, "substantially dissolved" means that the substrate is exposed on 90% or more of the surface. Here, in a case where the film and the substrate can be easily distinguished, the exposure ratio can be evaluated from the photograph of the appearance, and in a case where it is difficult to distinguish between the film and the substrate, the evaluation can be made by analysis using EDX or the like.

b) The dissolution property of dissolving in a Hank's balanced salt solution may satisfy one or both of the following iii) and iv), for example, when 2 mg of the protective film is immersed in the Hank's balanced salt solution:

iii) When immersed in 40 ml of Hank's balanced salt solution, the time when the concentration of the substance dissolved from the protective film becomes almost constant depending on the immersion time is 0.5 hours or more, preferably 1 to 72 hours, more preferably 2 to 48 hours after immersion;

iv) When immersed in 400 ml of Hank's balanced salt solution, the time when 2 mg of the protective film are substantially dissolved after immersion is 1 hour or more, preferably 2 to 672 hours, more preferably 3 to 336 hours, most preferably 4 to 168 hours.

The terms "substance that dissolves from the metal film" and "substantially dissolve" have the same definitions as above.

The metal film may be comprised of at least one metal selected from the group consisting of Mg, Ca, Zn and Fe. The protective film may be an alloy composed of two or more selected from these metals.

More, the metal film may further have a species selected from the group consisting of B, C, N and P. When the metal film is made of an alloy, the alloy may further comprise a species selected from the group consisting of B, C, N and P.

The metal film may comprise magnesium and optionally calcium, and calcium may have 0 to 40 % by weight, preferably 0.8 to 35 % by weight, more preferably 5 to 35 % by weight, further preferably 15 to 35 % by weight, most preferably 25 to 35 % by weight, where a total weight of magnesium and calcium is 100% by weight. The higher the calcium content, the larger the region of the amorphous structure. When the calcium content is 10% by weight, a sharp peak derived from crystals is observed, but when the calcium content is 20% by weight, a broad halo pattern derived from the amorphous structure is observed. A low-intensity peak that seems to be derived from microcrystals is observed in it, and at 25% by weight or more, almost no crystal-derived peaks are observed, and at 30% by weight, only a broad halo pattern derived from an amorphous structure is observed. When the biocompatible film of the present application comprises calcium, a large portion thereof has a substantially amorphous structure. As the portions of the amorphous structure increase, crystals such as $Mg_2Ca$ are difficult to form, and the biocompatible film dissolves uniformly in body fluids or simulated body fluids, facilitating uniform exposure of the hydrophilic surface of the substrate.

When the film is manufactured by sputtering, the content of calcium may be within 35 % by weight in order easily to manufacture the target.

The metal films of the present application may consist essentially of magnesium and optionally calcium. Preferably, the metal films of the present application may consist of magnesium and optionally calcium. Furthermore, the phrase "consisting of X" means that it is composed only of the components described in "X". Further, the phrase "consisting essentially of X" means that component(s) other than those described in "X" may include, but that the component(s) other than those described in "X" is comprised such that the component(s) does(do) not change the characteristics derived from the substance composed only of those described in "X".

More, in this case, the metal film may be free from $Mg_2Ca$. The term "be free from $Mg_2Ca$" means that a peak based on $Mg_2Ca$ is not observed in X-ray diffraction analysis, and preferably that a peak based on $Mg_2Ca$ is not observed at a diffraction angle at which diffraction peaks generated from crystals of the substrate and $Mg_2Ca$ do not overlap each other (the respective diffraction peaks are separated by 1° or more in X-ray analysis by using a cobalt (Co) tube lamp), for example, a peak is not observed in a range of 36 to 37° in X-ray analysis using a Co tube lamp.

The metal film may have an amorphous portion. The term "amorphous" means that no sharp peak is observed in X-ray diffraction analysis.

<<Layers other than biocompatible substrate and metal film>>

The biocompatible material according to the present application comprises the above-described biocompatible substrate; and the above-described metal film. However, the biocompatible material according to the present application may have other layers. For example, one or more layers for hydrophilization may be provided between the biocompatible substrate and the metal film. Examples of the layer may include, but are not limited to, an anodizing layer, a hydrothermal treatment layer, and the like for titanium or a titanium alloy. More, one or more layers may be provided on the upper portion of the above-mentioned film, that is, on the side opposite to the substrate.

The shape of the biocompatible material according to the present invention is not particularly limited, but for example, the biocompatible material may be one selected from the group consisting of a columnar shape, a cylindrical shape, a truncated cone shape, a conical shape, a shape having a screw-shaped threaded portion in a part of the shape, a rectangular parallelepiped and a cube, a block shape such as a rectangular parallelepiped and a cube having a partially inclined surface, and a wedge shape.

The application field of the biocompatible material according to the present invention is not particularly limited, but for example, the biocompatible material may be one selected from the group consisting of an artificial bone material, an intraosseous fixture material, a dental implant material, an orthodontic anchor screw material, a medullary nail material, and an interbody fixation material. Examples thereof may include, but are not limited to, an artificial bone, a pin, wire, a bolt, a screw, a washer, a medullary nail, and a vertebral body spacer.

<Method of producing the biocompatible material>

The present application provides a method for producing the above-described biocompatible material.

The method comprises the steps of:

(A) preparing a biocompatible substrate;
(B) hydrophilizing the surface of the biocompatible substrate; and
(C) forming a metal film on the surface of the biocompatible substrate;

thereby, to produce a biocompatible material comprising a biocompatible substrate having a hydrophilic surface; and a metal film provided on the surface of the substrate.

Furthermore, as the "biocompatible substrate", those described above can be used.

More, the "metal film" is not limited as long as it is a film that protects a hydrophilic substrate, but it may be the one described above.

The step (A) is a step of preparing a biocompatible substrate. The above-described "biocompatible substrate" may be commercially purchased, or the commercially purchased substrate may be formed into a desired shape. The method may comprise a step of grinding and/or polishing the surface of the purchased product or the obtained shape. Furthermore, as the grinding process and the polishing process, conventional processes may be used.

The step (B) is a step of hydrophilizing the surface of the biocompatible substrate.

The step (B) may be at least one selected from the group consisting of acid treatment, blasting treatment, anodizing, hydrothermal treatment, ultraviolet irradiation, plasma irradiation, laser irradiation, radiation irradiation, and ion irradiation.

In particular, the step (B) may be ion irradiation, and an integral power at the ion irradiation may be 0.4 W·min/cm$^2$ or more, preferably 0.8 W·min/cm$^2$ or more, more preferably 2.5 W·min/cm$^2$ or more, further preferably 5 W·min/cm$^2$ or more, most preferably 5 to 32 W·min/cm$^2$.

The step (C) is a step of forming a metal film on the surface of the biocompatible substrate, that is, on the hydrophilized surface.

The step (C) is not particularly limited as long as the metal film can be formed while maintaining the hydrophilicity of the hydrophilized surface.

The step (C) may comprise

(C1) preparing a sputtering target comprised of the metal film precursor; and
(C2) sputtering by using the sputtering target, to form the metal film on the biocompatible substrate obtained in the step (B).

In particular, the step (C1) may be (C1a) preparing the sputtering target comprised of magnesium and optional calcium, and

the step (C2) may be (C2a) sputtering by using the sputtering target and setting a temperature of the biocompatible substrate obtained in the step (B) at 130°C or lower, preferably 90°C or lower, more preferably 60°C or lower, to form the metal film comprised of magnesium and optional calcium on the biocompatible substrate, wherein the metal film comprises 0 to 40 % by weight of calcium where a total weight of magnesium and calcium is 100 % by weight.

A magnetron sputtering device may be used as the sputtering device.

In the magnetron sputtering device, a strong magnet (magnetron) is disposed behind a target, and sputtered particles (metal particles) generated by causing argon ions to collide with the target can be efficiently deposited on a substrate using a magnetic field. Upon sputtering, the sputtering voltage, the bias of the substrate, the pressure in the device, and the temperature of a substrate material can be adjusted, to form a film at a constant film formation rate.

The metal film based on magnesium has a linear expansion coefficient three times larger or more than that of zirconium, titanium, or a titanium alloy as the substrate. Thus, when the sputtering temperature is high, the temperature difference from around room temperature at which the implant is used and the sputtering temperature becomes large, and thus, tensile stress (thermal stress) is generated at the interface on the film side, and the film is easily peeled off. Therefore, in order to prevent the film from being peeled off after the film formation and to prevent harmful stress from remaining at the time of implanting the implant, the temperature of the substrate in sputtering is controlled to a certain temperature or less, that is, 130°C or less, preferably 90°C or less, more preferably 60°C or less, so that a film having high adhesiveness can be formed.

The above temperature can be estimated by calculating thermal stress as follows: When the temperature is lowered from the sputtering temperature to room temperature, the stress (thermal stress) at the interface generated can be approximately expressed by Equation 1, wherein $\Delta T$: a temperature difference between a substrate temperature during sputtering Td and room temperature Tr, $\alpha_1$: an average linear expansion coefficient of the substrate between the temperatures Tr and Td, $\alpha_2$: an average linear expansion coefficient of the coating film between the temperatures Tr and Td, $E_1$: an average elastic modulus of the substrate between the temperatures Tr and Td, and $E_2$: an average elastic modulus of the film between the temperatures Tr and Td.

$$\sigma = \frac{E_1 E_2 (\alpha_1 - \alpha_2) \Delta T}{E_1 (1 - \alpha_2 \Delta T) + E_2 (1 - \alpha_1 \Delta T)} \qquad \text{(Equation 1)}$$

For example, assuming that the substrate is zirconia, and the coating film is pure magnesium, calculation is performed by applying the linear expansion coefficients as $8 \times 10^{-6}$ and $25 \times 10^{-6}$ and the elastic moduli as 210 GPa and 40 GPa, respectively.

Since the yield strength of pure magnesium is generally said to be about 90 to 100 MPa, the shear yield strength is about 50 MPa. In order to suppress the thermal stress to at least this value or less, the temperature difference may be 100 degrees or less. For example, when the operating temperature is 36°C as a body temperature, the film formation temperature may be 130°C or lower, and is preferably 90°C or lower, which is twice the safety factor or 60°C or lower in consideration of about three times the safety factor.

The method according to the present invention may comprise the step of (D) ion cleaning the surface of the biocompatible substrate after the step (B) and before the step (C). The step of ion cleaning is a step in which impurities on the surface are removed at the atomic level by bombarding the surface of the substrate with argon ions or the like while appropriately adjusting the bias in a vacuum, specifically in a vacuum chamber. By performing the process appropriately, the adhesion of the metal film can be stabilized and the surface of the substrate can be activated.

The method according to the present invention may comprise a step(s) other than the above-described steps (A) to (D). For example, as described above, after step (A) and before step (B), the method may comprise the step of forming the "biocompatible substrate" into a desired shape and the step of grinding and/or polishing the surface of the shape.

For example, when a layer(s) is(are) provided between the substrate and the metal film, the step of providing the layer(s) may be provided after step (B) and before step (C) .

Hereinafter, the present invention is specifically described with reference to, but is not limited only to, examples.

Examples

<Substrate Material>

As a substrate material assumed to be an implant, smooth plate zirconia A-F and A-F2 cut into 10 × 10 mm with a thickness of 3 mm and subjected to general grinding processing on one plane surface were used. A rough plate zirconia A-R was used, one surface of which was roughened by forming micro lattice-like grooves on the surface. Furthermore, a glass substrate B having a dimension of 26 mm × 76 mm with a thickness of 1.2 mm was also used to examine the details of the characteristics of the formed metal film.

The surface roughness of each sample was measured in accordance with JIS B0601:2013, with a reference length of 0.8 mm, an evaluation length of 4.0 mm, and cutoff values $\lambda c$ and $\lambda s$ of 0.8 mm and 0.25 um, respectively. In addition, in order to equalize the influence of the grinding direction, the surface roughness was measured in two directions parallel and perpendicular to the grinding direction and averaged. Table 1 summarizes the surface roughness values for each sample.

Table 1. Surface roughness of each substrate material

|  | Ra ($\mu$m) | Rz ($\mu$m) |
|---|---|---|
| Smooth plate zirconia (A-F) | 0.20 | 1.29 |

(continued)

|  | Ra ($\mu$m) | Rz ($\mu$m) |
| --- | --- | --- |
| Smooth plate zirconia (A-F2) | 0.35 | 1.91 |
| Rough plate zirconia (A-R) | 10.34 | 31.94 |

<Ion irradiation process>

Ion irradiation in a vacuum chamber was used to form a hydrophilic surface. In the ion irradiation process, first, the pressure was reduced to a predetermined value, harmful gases were removed from the chamber, and then argon gas was sealed. By appropriately adjusting the voltage required for discharge and the substrate bias, ion irradiation process was carried out under five conditions, integral powers (power W x time (min)/cross-sectional area ($cm^2$)) of: 0 (untreated), 0.495, 0.99, 2.8 and 5.36 W·min/$cm^2$.

<Sputtering process>

Sputtering process was carried out in the same vacuum chamber following the ion irradiation process. As a sputtering target, pure magnesium, and four kinds of ingots manufactured by dissolving pure magnesium and pure calcium at a predetermined ratio were machined into a disk shape having a diameter of about 120 mm and used. The four kinds of ingots were those in which the amount of calcium was 0% (Mg), 10% (Mg10%Ca), 20% (Mg20%Ca), and 30% (Mg30%Ca), and the remaining amount was magnesium. The % of the calcium amount is a ratio of the calcium weight when the total of the calcium weight and the magnesium weight is 100 % by weight and is represented by wt% as below.

Calcium wt% = Calcium Weight / (Magnesium Weight + Calcium Weight) $\times$ 100

Furthermore, targets with a calcium amount of 40% by weight (Mg40%Ca) or 50% by weight (Mg50%Ca) were tried to make in a manner similar to the target with Mg10%Ca and the like, by using pure magnesium and pure calcium. However, the targets could not be used since cracks occurred during machining and since they were partially damaged.
The substrate was disposed on the stage of the sputtering device so as to face the sputtering target. Furthermore, the plate-shaped substrate was disposed such that the ground surface or rough surface was the upper surface.
The temperature of the substrate was kept at room temperature, the pressure of argon was 1 to 10 mTorr, the sputtering voltage and the bias of the substrate were adjusted, and the time of the film formation process (deposition) was adjusted, smooth plate zirconia (A-F), rough plate zirconia A-R and glass substrate B were placed in the same chamber, and then the film formation process was carried out.
Table 2 shows each film thickness and weight of magnesium alone film (Mg film), a film with a calcium amount of 20% by weight (Mg20%Ca film), and a film with a calcium amount of 30% by weight (Mg30%Ca film), each of which was obtained by sputtering by using each target of magnesium alone (Mg), 20% by weight of calcium (Mg20%Ca) and 30% by weight of calcium (Mg30%Ca) after ion irradiation process at an integral power of 0.99 W·min/$cm^2$. The thickness of the obtained film was measured by using a sample sputtered onto glass substrate B treated in the same chamber as each substrate. That is, the thickness of the obtained film was measured by actually measuring a gap distance by a stylus method, the gap distance being between a portion on the substrate where the film was formed and another portion where the film was not formed by performing the above-described masking. Further, the weight of the film was calculated by subtracting the weight of the substrate A-F, A-R or A-F2 before sputtering process from the weight of the sample after sputtering each film.
X-ray diffraction analysis was carried out for each coated film by using an X-ray diffractometer (D8 ADVANCE, manufactured by Bruker Corporation) under the conditions of a detector: a two-dimensional detector, tube lamp: Co, tube lamp voltage: 30 kV, tube lamp current: 40 mA, slit: $\Phi$ 1.0 mm, and collimator: $\Phi$ 1.0 mm.
Fig. 1 shows X-ray diffraction profiles of a magnesium alone film (Mg film), a film with a calcium amount of 20% by weight (Mg20%Ca film) and a film with a calcium amount of 30% by weight (Mg30%Ca film), each of which was obtained by sputtering each target of magnesium alone (Mg), 20% by weight of calcium (Mg20%Ca) and 30% by weight of calcium (Mg30%Ca) after ion irradiation process at an integral power of 0.99 W·min/$cm^2$.
Fig. 1 shows that a sharp and strong peak derived from crystal is observed around $2\theta$ = 40 degrees derived from the (0 0 -2) plane of magnesium for the Mg film and the Mg10%Ca film. Comparing the peak intensities of the Mg film and the Mg10%Ca film, the peak intensity of the Mg10%Ca film is significantly lower, indicating that the crystallinity is lowered. In addition, in the Mg20%Ca film, a low-intensity peak thought to be derived from microcrystals was observed in the broad halo pattern derived from the amorphous structure, and in the Mg30%Ca film, only the broad halo pattern derived from the amorphous structure was observed.

These results show that the Mg film is crystalline, that the Mg10%Ca film is crystalline with low crystallinity, that the Mg20%Ca film has a mixed structure of microcrystals and amorphous, and that the Mg30%Ca film is non-crystalline. In general, when the amount of calcium added to magnesium exceeds 0.8% by weight, calcium does not form a solid solution in magnesium and $Mg_2Ca$ precipitates. However, the presence of intermetallic compounds such as $Mg_2Ca$ was not observed in any of the films. Moreover, although not shown, no difference was observed in the film structure detected due to the difference in ion irradiation intensity.

Table 2. Thickness and weight of each film

| Substrate | Film Composition | Film thickness measured by glass substrate B ($\mu$m) | Film weight (mg) |
|---|---|---|---|
| A-F | Mg | 10.482 | 2.9 |
| | Mg20%Ca | 10.193 | 3.0 |
| | Mg30%Ca | 10.144 | 2.6 |
| A-R | Mg | 10.482 | 2.5 |
| | Mg20%Ca | 10.193 | 2.7 |
| | Mg30%Ca | 10.144 | 2.6 |
| A-F2 | Mg | 5.282 | 1.1 |
| | Mg20%Ca | 4.875 | 1.2 |
| | Mg30%Ca | 4.957 | 1.4 |

<Hydrophilicity>

About 5 $\mu$l of pure water was dropped on the substrate or film, and the extent to which the water drops were rounded due to surface tension was photographed with a CCD camera from a direction perpendicular to the dropping surface. The general $\theta/2$ method was used to measure the angle between the solid surface and the straight line connecting the left and right endpoints of the droplet and the apex of the droplet, and to double the angle, to obtain the water droplet contact angle between the liquid surface and the solid surface. In addition, since water droplets tended to spread along the polishing direction of the substrate, the contact angle was measured in a direction parallel to the polishing direction of the substrate and in a direction perpendicular to the polishing direction of the substrate. The water droplet contact angle was obtained by measuring from two directions of the case and averaging them.

For the smooth plate zirconia A-F and A-F2, the rough plate zirconia A-R, and the glass substrate B, the water droplet contact angle was measured as a hydrophilicity evaluation after ion irradiation process. Fig. 2 shows water droplet contact angles of surfaces of smooth plate zirconia A-F, rough plate zirconia A-R, and glass substrate B irradiated with ions at an integral power of 0, 0.495, 0.99, 2.85 or 5.36 W·min/cm$^2$.

Further, after the ion irradiation process, sputtering process was performed to protect the ion-irradiated surface with a metal film. After the sample coated with the metal film was wrapped in medicine wrapping paper and stored in the atmosphere for two months, the water droplet contact angle of the substrate was measured immediately after removing the metal film. In order to remove the metal film, the substrate was immersed and shaken in 50 ml of a 5% hydrochloric acid aqueous solution for about 10 seconds to dissolve the film until the entire surface of the substrate was exposed. After additional cleaning by immersing and shaking in another ultrapure water for about 10 seconds, it was dried by blowing with argon gas.

Table 3 shows water drop contact angles for smooth plate zirconia A-F and rough plate zirconia A-R, each of which was subjected to hydrophilization treatment by ion irradiation at an integral power of 0.495, 0.99, or 5.36 W·min/cm$^2$, and stored for two months after the treatment.

Further, Table 3 shows water drop contact angles for smooth plate zirconia A-F and rough plate zirconia A-R immediately after removing Mg30%Ca films in the above-described process after two months of protection by the films for smooth plate zirconia A-F and rough plate zirconia A-R, each of which was subjected to hydrophilization treatment by ion irradiation at an integral power of 0.495, 0.99, or 5.36 W·min/cm$^2$, and coated and protected with Mg30%Ca film by sputtering process.

Table 4 shows water drop contact angles for smooth plate zirconia A-Fs immediately after removing films in the above-described process after two months of protection by the films for smooth plate zirconia A-Fs, each of which was subjected to hydrophilization treatment by ion irradiation at an integral power of 0.99 W·min/cm$^2$, and coated and protected with Mg film, Mg20%Ca film, or Mg30%Ca film by sputtering process.

More, water droplet contact angles were measured immediately after removing, by the above-mentioned steps, the metal films, each of which was wrapped in medicine wrapping paper and stored in the atmosphere within 1 week, for 1 month, 2 months, 4 months, 7 months and 15 months immediately after coating by sputtering.

Fig. 3 shows water droplet contact angles immediately after removing, by the above-mentioned steps, the Mg30%Ca films, each of which was prepared by ion irradiation at an integral power of 0.99 W·min/cm$^2$, followed by sputtering, coated on smooth plate zirconia A-F, and stored within one week, 1 month, 2 months, 4 months, 7 months, and 15 months after the coating.

Further, Fig. 3 shows water droplet contact angles immediately after removing, by the above-mentioned steps, the Mg30%Ca films, each of which was prepared by ion irradiation at an integral power of 0.99 W·min/cm$^2$, followed by sputtering, coated on smooth plate zirconia A-F2, and stored within one week, 1 month, 2 months, 4 months, 7 months, and 15 months after the coating.

More, Fig. 3 shows water droplet contact angles immediately after removing, by the above-mentioned steps, the Mg30%Ca films, each of which was prepared by ion irradiation at an integral power of 0.99 W·min/cm$^2$, followed by sputtering, coated on rough plate zirconia A-R, and stored within one week, 1 month, 2 months, 4 months, 7 months, and 15 months after the coating.

Table 3. Water droplet contact angles after 2 months after hydrophilization treatment (depending on integral power of ion irradiation)

| Substrate roughness | | Smooth plate (A-F) | | | Rough plate (A-R) | | |
|---|---|---|---|---|---|---|---|
| Intensity of hydrophilization treatment (Integral power of ion irradiation (W ·min/cm$^2$) ) | | 0.495 | 0.99 | 5.36 | 0.495 | 0.99 | 5.36 |
| Water droplet contact angle (°) | Hydrophilization treatment + 2 months | 96.6 | 93.6 | 98.6 | 109.5 | 109.6 | 118.0 |
| | Hydrophilization treatment + Metal film + 2 months + Removing the film | 16.2 | 11.1 | 7.2 | 17.1 | 9.2 | 5.9 |

Table 4. Water droplet contact angles after 2 months after hydrophilic treatment (depending on kinds of metal films)

| Substrate roughness | Smooth plate (A-F) | | |
|---|---|---|---|
| Kinds of metal films | Mg film | Mg20%Ca film | Mg30%Ca film |
| Water droplet contact angle (°) | 15.1 | 11.2 | 11.1 |

From Figs. 2 and 3, and Tables 3 and 4, the following can be understood.

For example, in dental implant materials, it is important to directly bond the bone and the dental implant material in order to prevent loosening of the screw part, and it is effective to improve the hydrophilicity of the surface of the screw part material. Further, in order to prevent periflammation in the abutment, it is important not to allow bacteria to adhere to it, and to do so, it is necessary to reduce the hydrophilicity of the surface of the material for the abutment.

Fig. 2 shows that the water droplet contact angle of rough zirconia that has not been subjected to ion irradiation is 70° or more at maximum, while the water droplet contact angle of rough zirconia that has been subjected to ion irradiation is 5° or less at the lowest, which exhibits superhydrophilicity. Further, Fig. 2 shows that in smooth zirconia with a lower substrate surface roughness, the difference in water droplet contact angles before and after ion irradiation is smaller. Therefore, by appropriately combining the substrate surface roughness and the presence or absence of ion irradiation or the conditions thereof, it is possible to provide the water droplet contact angle required for each implant site.

Table 3 shows that the water droplet contact angles of smooth and rough zirconias stored for two months after the hydrophilization treatment by ion irradiation become 110° or more at maximum, while the water droplet contact angles of smooth and rough zirconias, each of which was subjected to hydrophilization treatment by ion irradiation, coated with the metal film, and stored for two months, followed by removing the metal film become 6° or less at minimum exhibiting superhydrophilicity. Therefore, Table 3 shows that the hydrophilic surface of the substrate imparted by ion irradiation becomes more hydrophilic in the step of coating with the metal film, and that the hydrophilic surface is protected by the metal film. Furthermore, it can be seen from <Dissolution rate test using simulated body fluid> described later that the metal film dissolves in vivo. Therefore, it can be seen that the metal film can dissolve in vivo and that the hydrophilic surface is exposed.

Table 4 shows that any one of water droplet contact angles of the smooth zirconias, each of which was subjected to the hydrophilization treatment by ion irradiation, coated with three kinds of metal films with different chemical components and structures, followed by removing the metal films is 20 ° or less, which exhibits highly hydrophilicity.

Therefore, it can be seen that the hydrophilic surface can be protected regardless of the type of metal film.

Further, the protective performances of the Mg20%Ca film and the Mg30%Ca film each having an amorphous structure are slightly superior to that of the Mg film (magnesium only film) having crystalline.

Therefore, use of a metal film having an amorphous structure can exhibit superior protection performance.

Fig. 3 shows that the water droplet contact angles of smooth zirconia, which was subjected to the hydrophilization treatment by ion irradiation, coated by sputtering, and stored in the atmosphere for 15 months, followed by removing the metal film, was maintained at 30° or less. Fig. 3 also shows that long-term exposure to the atmosphere increases the water droplet contact angles on the metal film, but maintains the hydrophilicity of the hydrophilic surface under the film.

Therefore, by coating with a metal film, it is possible to store it in the atmosphere for a long period of time without using a special storage container.

<Dissolution rate test using simulated body fluid>

A dissolution rate test in body fluid was performed for the samples, each of which was subjected to the ion irradiation to smooth plate zirconia A-F2 at an integral power of 0.99 W·min/cm$^2$, and coated with a Mg film, a Mg20%Ca film, and a Mg30%Ca film by sputtering. For the samples, the sputtered film was attached to the top and side surfaces of the sample, but not to the bottom surface.

Hank's balanced salt solution (HBSS(-) solution (phenol red free), Fuji Film Wako Pure Chemical) containing no calcium or magnesium was used as the simulated body fluid.

The volume of the simulated body fluid was 40 ml or 400 ml.

Therefore, assuming that the apparent surface area ignoring the influence of surface roughness is 220 mm$^2$, the simulated body fluid volume per unit area of the film in contact with the solution was 0.182 ml/mm$^2$ or 0.0182 ml/mm$^2$, respectively.

Dissolution rates were evaluated from pH measurements using the glass electrode method. A desktop pH tester (HORIBA, F-74) and a GRT composite electrode (9615S-10D, HORIBA) were used for pH measurement.

The dissolution rate test was performed by immersing the sample in 40 ml of HBSS (-) solution kept at 37° in an air bath type constant temperature device, and the pH was measured after 1 hour, 3 hours, 6 hours, 12 hours and 24 hours. In addition, after 6 hours, 24 hours and 168 hours after immersion in 400 ml of HBSS (-) solution kept at 37° in an air bath type constant temperature device, it was taken out, and the exposed surface was identified and the thickness of the obtained film was measured be an XRD device ((D8ADVANCE, BRUKER). SEM EDX (JSM5900LVM, JEOL) was used to identify the exposed surface.

Fig. 4 shows the results of pH measurement as a dissolution rate evaluation in HBSS (-) immersion for smooth zirconia A-F2s, each having a Mg film, a Mg20%Ca film and a Mg30%Ca film.

Further, the calculated values of the amount of magnesium and calcium components per unit volume mol/cm$^3$ in the Mg film, the Mg20%Ca film and the Mg30%Ca film when the film thickness is 5.28 um and the total cross-sectional area is 220 mm$^2$ are shown in Table 5.

Fig. 5 shows the XRD measurement results of smooth zirconia A-F2 with a Mg film before immersion in HBSS(-) and after immersion for 6 hours, 24 hours and 168 hours, respectively.

Further, based on the results of Fig. 5, the ratio of the diffraction intensity Im from the (0 0 -2) plane of the magnesium film and the diffraction intensity Iz from the (1 0 -1) plane of the zirconia substrate was calculated. Table 6 shows the value obtained by subtracting the Im/Iz after 168 hours from the peak intensity ratio Im/Iz obtained from the peak intensity ratio Im/Iz, and the estimated value of the film thickness based on the relationship between the intensity ratio and the value of the film thickness T after 0 hour.

If the relationship between immersion time and film thickness is approximated by an exponential function, the change in film thickness with immersion time can be roughly predicted by the following equation.

$$dT/dt = -1.39 \exp(-0.15t)$$

Table 5. Calculated values of magnesium and calcium existed in the films

| Kinds of film | Mg film | Mg20%Ca film | Mg30%Ca film |
|---|---|---|---|
| Amount of Mg (mol/cm$^3$) | 0.0716 | 0.0559 | 0.0483 |
| Amount of Ca (mol/cm$^3$) | 0 | 0.0084 | 0.0126 |

Table 6. Immersion time of the film in simulated body fluid, Ratio of intensities Im/Iz, and Assumed thickness T

| Item | Immersion time in simulated body fluid (HBSS(-)) | | | |
|---|---|---|---|---|
| | After 0 hour | After 6 hours | After 24 hours | After 168 hours |
| Ratio of intensities Im/Iz | 2.16 | 1.07 | 0.14 | 0 |
| Assumed thickness T ($\mu$m) | 5.28 (observed) | 2.93 | 0.66 | 0 |

Fig. 6 shows appearance photographs of smooth zirconia A-F2 with a Mg30%Ca film before immersion in HBSS (-), taken out after 6 hours immersion and 168 hours immersion, and analysis results by EDX of the surface of the sample taken out after 6 hours immersion.

In addition, Table 7 shows the identification results obtained from Fig. 6. The sample taken out after 6 hours immersion was covered with a metallic color layer, black or white layer, and there were almost no areas where polishing scratches on the substrate could be observed, and the surface of the biocompatible substrate was not exposed. In the sample taken out after 24 hours immersion, almost no metal-colored layer was observed, the portions where polishing scratches on the substrate were observed increased, and the exposed portions in surface of the biocompatible substrate increased. More, in the sample after 168 hours immersion, polishing scratches were observed over the entire surface of the substrate, and the surface of the biocompatible substrate was exposed over the entire surface.

Table 7. Appearances of the surfaces of the samples after immersion in the simulated body fluid and identification results

| Appearance By photograph | Results of EDX analysis | Identification |
|---|---|---|
| Metallic color layer | Mg-richer and Ca-richer | Portions where Mg30%Ca film was remained |
| Black or white layer | Mg-richer and O-richer | Portions where compositions in the film were converted into oxide (s) or hydroxide(s) during the immersion |
| Portions where polishing scratches of the substrate are observed (white colored substrate) | Zr-richer | Portion where the substrate (zirconia) is exposed |

Figs. 4, 5 and 6, and Tables 5, 6 and 7 show the following: For example, after a dental implant is implanted in the jawbone, occlusion with dentures cannot be performed until sufficient osseointegration occurs. Therefore, there is a demand for early osteosynthesis of dental implants. Fig. 4 and Table 5 show that after immersion in the simulated body fluid, the metal film is ionized immediately after immersion, resulting in a sudden change in pH, reaching a pH of 9.5 or higher at maximum after 24 hours.

Therefore, Fig. 4 and Table 5 show that dissolution of the film occurs early after implantation in the bone.

Fig. 5 and Table 6 show that the film thickness rapidly decreases immediately after immersion, that the film thickness hardly changes after 24 hours of immersion, and that all of the metal film dissolves within 168 hours.

Fig. 6 and Table 7 show that the substrate was hardly exposed after 6 hours immersion, but that the substrate was gradually exposed thereafter, and that the substrate was exposed on all sides at least within 168 hours immersion.

Therefore, after a dental implant wherein the hydrophilic surface is protected by a metal film is implanted, the film dissolves early, exposing the activated surface, thereby promoting early osseointegration.

<In vitro biological reaction test using the simulated body fluid>

An in vitro bioreaction test using a simulated body fluid was carried out for the samples, each of which was obtained by ion irradiation to smooth plate zirconia A-F and rough plate zirconia A-R at integral powers of 0.495, 0.99 and 5.36 W·min/cm$^2$, followed by sputtering, to form Mg30%Ca.

A Hank's balanced salt solution containing calcium and magnesium (HBSS(+) solution) was used as the simulated body fluid. In order to bring the equilibrium pH of the solution closer to that of the in vivo environment, the amount of the HBSS(+) solution was adjusted so that the equilibrium pH after elution of the sputtering film was 8.0 or less. For comparison, substrates, each of which was not subjected to ion irradiation and sputtering, were tested by immersing them in two kinds of solutions, HBSS(+), and HBSS(+) after dissolution of Mg30%Ca film.

In order to carry out a simulated body fluid immersion test, each sample was immersed in 500 ml of simulated body fluid, held in a constant temperature bath at 37° C for one week, and then taken out of the fluid.

Fig. 7 shows a photograph of smooth plate zirconia A-F, a photograph of a sample in which smooth plate zirconia A-F was irradiated with ions at an integral power of 5.36 W·min/cm$^2$, followed by sputtering to coat with a Mg30%Ca film, and a photograph of a sample in which a smooth plate zirconia A-F was irradiated with ions at an integral power of 5.36 W·min/cm$^2$, followed by sputtering to coat with a Mg30%Ca film, immersed in a simulated body fluid (HBSS(+)), and then taken out after one week. Further, although not shown, it can be seen that the state of the surface on which the film was formed was observed over time, and that as a result, a new layer was formed on the surface after all of the film formed was dissolved into the fluid in about four days. X-ray diffraction (XRD) analysis and fluorescent X-ray analysis (EDX) for the structure and components of the newly formed layer revealed that there is a high possibility that bone-like apatite (HAp) is formed. It is assumed that this is because the deposition of HAp on the substrate was promoted by the dissolution of magnesium and calcium, which are components of the film.

Table 8 shows calculated values by image analysis of the samples, each of which was obtained by applying ion irradiation to smooth plate zirconia A-F at integral powers of 0.495, 0.99 and 5.36 W·min/cm$^2$, respectively, followed by sputtering to coat with a Mg30%Ca film, immersing in HBSS(+) for one week, and taking out of the HBSS(+). The image analysis was carried out by taking a photograph of the obtained sample by a microscope (VHX7000, KEYENCE) from a direction tilted 15 degrees from the axis perpendicular to the sample, and by image analyzing the metal film remaining portion, the area fraction of the portion not covered with HAp while the base material is exposed, and the HAp coverage based on the image obtained by synthesizing the matching portions. Fig. 8 is an image used when calculating the area fractions of the integral powers of 0.495 and 5.36 W·min/cm$^2$ for the smooth materials (A-F) among the samples in Table 8.

When another sample at the integral power of 0.495 or 0.99 was immersed in HBSS(+) for 3 weeks and taken out, no residual metal film was observed. On the other hand, the area fraction of the portion where the base material was exposed and was not covered with HAp was found to be approximately the same as that when immersed in HBSS(+) for one week.

Also, with respect to the substrate which was not sputtered after ion irradiation, formation of HAp was not observed in either of the two types of solution immersion, HBSS(+) and HBSS(+) after dissolution of the metal film.

Table 8. Coverage ratio of Hap formed after HBSS(+) immersion

| Surface roughness of substrate | Smooth plate (A-F) | | |
|---|---|---|---|
| Integral power of ion irradiation (W·min/cm$^2$)) | 0.495 | 0.99 | 5.36 |
| Area fractions where the metal film was remained Ar (%) | 9.4% ($\pm$4.2%) | 5.4% ($\pm$1.0%) | 0% |
| Area fractions where the substrate was exposed and not covered with HAp $A_n$ (%) | 2.4% ($\pm$1.7%) | 1.7% ($\pm$0.8%) | 0.1% ($\pm$0.1%) |
| Hap coverage ratio $A_{HAp}$ (%) $A_{HAp} = 100 - (A_r + A_n)$ | 88.2% | 92.9% | 99.9% |

Figs. 7 and 8, and Table 8 show the following:
For example, when bone apatite is formed in vivo on the surface of a dental implant material, osteosynthesis is performed via the bone apatite, and the dental implant can be strongly osseointegrated.

Fig. 7 shows that apatite was abundantly formed on the entire surface of zirconia whose hydrophilic surface was maintained by the metal film after ion irradiation, but that no apatite was formed on zirconia whose hydrophilic surface was not maintained by the metal film after ion irradiation.

Table 8 shows that when the degrees of hydrophilization by the integral power of the ion irradiation were different, the HAp coverage ratio also varied among the samples in which the hydrophilicity was maintained by the metal film. Further, samples exhibiting higher hydrophilicity exhibit higher HAp coverage. A surface activated by hydrophilization has a higher energy state. Apatite was formed on the zirconia with the hydrophilic surface exposed in the solution, while apatite was not formed on the non-hydrophilized zirconia, suggesting that an exposure of activated surface having a higher energy state in the fluid forms a large amount of nuclei of apatite, resulting in the formation of abundant apatite over the entire surface.

Therefore, use of a dental implant that has undergone a hydrophilization treatment that exhibits high hydrophilicity and whose hydrophilic surface is protected by a metal film spontaneously exposes the activated surface in vivo, promotes the formation of apatite on the entire surface, and can encourage bone formation earlier.

**Claims**

1. A biocompatible material comprising a biocompatible substrate having a hydrophilic surface; and a metal film provided on the surface of the substrate, the metal film having the dissolution property of dissolving in body fluid or simulated body fluid.

2. The biocompatible material according to claim 1, wherein when the biocompatible material is brought into contact with the body fluid or simulated body fluid, the metal film dissolves and the biocompatible substrate having the hydrophilic surface is exposed.

3. The biocompatible material according to claim 1, wherein the bodily fluid is at least one selected from the group consisting of blood, lymph, bone marrow fluid, and tissue fluid, and the simulated bodily fluid is at least one selected from the group consisting of physiological saline, phosphate-buffered saline (PBS), Hank's balanced salt solution (HBSS), SBF solution, plasma solution, cell culture solution, Eagle (MEM) solution, DMEM solution, and serum medium.

4. The biocompatible material according to claim 1, wherein the simulated bodily fluid is Hank's balanced salt solution.

5. The biocompatible material according to claim 1, wherein the hydrophilic property has a water droplet contact angle of 90° or less.

6. The biocompatible material according to claim 1, wherein the metal film comprises at least one metal selected from the group consisting of Mg, Ca, Zn and Fe.

7. The biocompatible material according to claim 1, wherein the metal film comprises magnesium and optionally calcium, and calcium has 0 to 40 % by weight, where a total weight of magnesium and calcium is 100 % by weight.

8. The biocompatible material according to claim 7, wherein the metal film is free from $Mg_2Ca$.

9. The biocompatible material according to claim 1, wherein the metal film has an amorphous portion.

10. The biocompatible material according to claim 1, wherein the substrate is at least one selected from the group consisting of pure titanium, a cobalt-chromium alloy, stainless steel, titanium alloys, zirconia, alumina, calcium phosphate and magnesia.

11. The biocompatible material according to claim 1, wherein surface roughness Ra of the substrate is 50 um or less.

12. The biocompatible material according to claim 1, wherein a shape of the biocompatible material is one selected from the group consisting of a cylindrical shape, a truncated cone shape, a conical shape, a shape having a screw-shaped threaded portion in a part of the shape, a rectangular parallelepiped and a cube, a block shape having a partially inclined surface, and a wedge shape.

13. The biocompatible material according to claim 1, wherein the biocompatible material is one selected from the group consisting of an artificial bone material, an intraosseous fixture material, a dental implant material, an orthodontic anchor screw material, a medullary nail material, and an interbody fixation material.

**14.** A method of producing a biocompatible material, comprising the steps of:

(A) preparing a biocompatible substrate;
(B) hydrophilizing the surface of the biocompatible substrate; and
(C) forming a metal film on the surface of the biocompatible substrate;

thereby, to manufacture the biocompatible material comprising a biocompatible substrate having a hydrophilic surface; and a metal film provided on the surface of the substrate.

**15.** The method according to claim 14, wherein the step (C) comprises

(C1) preparing a sputtering target comprised of the metal film precursor; and
(C2) sputtering by using the sputtering target, to form the metal film on the biocompatible substrate obtained in the step (B).

**16.** The method according to claim 15, wherein the step (C1) is (C1a) preparing the sputtering target comprised of magnesium and optional calcium, and
the step (C2) is (C2a) sputtering by using the sputtering target and setting a temperature of the biocompatible substrate obtained in the step (B) at 130°C or lower, to form the metal film comprised of magnesium and optional calcium on the biocompatible substrate, wherein the metal film comprises 0 to 40 % by weight of calcium where a total weight of magnesium and calcium is 100 % by weight.

**17.** The method according to claim 14, wherein the step (B) is at least one selected from the group consisting of acid treatment, blasting treatment, anodizing, hydrothermal treatment, ultraviolet irradiation, plasma irradiation, laser irradiation, radiation irradiation, and ion irradiation.

**18.** The method according to claim 14, wherein the step (B) is ion irradiation, and an integral power at the ion irradiation is 0.4 W·min/cm$^2$ or more.

**19.** The method according to claim 14 further comprising the step (D) of ion cleaning the surface of the biocompatible substrate after the step (B) and before the step (C).

Fig. 1

Fig. 2

Fig. 3

Fig. 4

Fig. 5

| Before immersion | After 6 hours immersion | After 168 hours immersion |

Fig. 6

Before coating

After coating

After immersion in simulated body fluid

Fig. 7

Portion where substrate is exposed and HAp is not formed

Portion where
metal film is
remained

Ion irradiation at integral power of
0.495 Wmin/cm²

Ion irradiation at integral power of
5.36 Wmin/cm²

Fig. 8

## INTERNATIONAL SEARCH REPORT

| | International application No. |
|---|---|
| | **PCT/JP2022/032506** |

**A. CLASSIFICATION OF SUBJECT MATTER**

*A61L 27/04*(2006.01)i; *A61L 27/06*(2006.01)i; *A61L 27/12*(2006.01)i; *A61L 27/30*(2006.01)i; *A61L 27/42*(2006.01)i; *A61L 27/50*(2006.01)i
FI: A61L27/04; A61L27/42; A61L27/50; A61L27/06; A61L27/12; A61L27/30

According to International Patent Classification (IPC) or to both national classification and IPC

**B. FIELDS SEARCHED**

Minimum documentation searched (classification system followed by classification symbols)

A61L27/04; A61L27/06; A61L27/12; A61L27/30; A61L27/42; A61L27/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2012-196461 A (YOU&I CORP.) 18 October 2012 (2012-10-18) | 1-17, 19 |
| | examples 1, 2, 4, 5, paragraph [0047] | |
| Y | | 1-17, 19 |
| A | | 18 |
| X | PARK, Ki-Deog et al., Effect of magnesium and calcium phosphate coatings on osteoblastic responses to the titanium surface, J. Adv. Prosthodont., 2013, vol. 5, pp. 402-408 | 1-17, 19 |
| | p. 403, column of "RF magnetron sputtering", table 1, p. 402, right column, lines 1-3 | |
| Y | | 1-17, 19 |
| A | | 18 |

☐ Further documents are listed in the continuation of Box C.     ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **04 November 2022** | **22 November 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

| International application No. |
| --- |
| **PCT/JP2022/032506** |

| Patent document cited in search report | | | Publication date (day/month/year) | Patent family member(s) | Publication date (day/month/year) |
| --- | --- | --- | --- | --- | --- |
| JP | 2012-196461 | A | 18 October 2012 | US 2010/0075162 A1 examples 1, 2, 4, 5, paragraph [0066] EP 2063816 A1 KR 10-2008-0027202 A CN 101516292 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014193249 A
- JP 2016154935 A
- JP 2017169821 A
- JP 2016053195 A
- JP 2007181515 A
- JP 2014014487 A
- JP 6095653 B
- JP 6154806 B
- JP 5705163 B